# EUROPEAN PATENT APPLICATION

(11) **EP 3 093 298 A1**
(43) Date of publication of application: **16.11.2016**
(21) Application number: 15733139.8
(22) Date of filing: 06.01.2015
(51) Int. Cl.: C08F 2/00, C08F 20/10, C08F 2/10, C08J 3/075, C08J 3/24

(54) **METHOD FOR PREPARING SUPERABSORBENT POLYMER**

(30) Priority: 06.01.2014 KR 20140001259
(71) Applicant: Hanwha Chemical Corporation, Seoul 100-797 (KR)
(72) Inventor: SIM, Yu Jin, Daejeon 305-720 (KR); KIM, Eui Duk, Daejeon 305-720 (KR); KIM, Ji Yeon, Dalseong-gun Daegu 711-765 (KR); PAIK, Choong Hoon, Daejeon 305-720 (KR); OH, Seok Heon, Daejeon 305-720 (KR); LEE, Min Ho, Pohang-si Gyeongsangbuk-do 791-786 (KR); CHOI, Dae Keon, Jeonju-si Jeollabuk-do 560-792 (KR)
(74) Representative: Glawe, Delfs, Moll
(86) International application number: PCT/KR2015/000091
(87) International publication number: WO 2015/102463

(57) **Abstract**

The present invention relates to a method for preparing a superabsorbent polymer. The method for preparing a superabsorbent according to the present invention comprises the steps of: polymerizing a monomer composition into a superabsorbent polymer in a polymerization reactor; grinding the obtained superabsorbent polymer; and hydrolyzing the fine particles generated during the process and reusing the same for the monomer composition.

## Description

### TECHNICAL FIELD

The present invention relates to a method for preparing a superabsorbent polymer.

### BACKGROUND ART

Superabsorbent polymers (SAPs) are synthetic polymer materials having a function of absorbing moisture of 500 to 1000 times their own weight, and have been differently named as superabsorbent material (SAM), absorbent gel material (AGM), and the like depending on developers. Such superabsorbent polymers have been widely used as materials in the fields of water retaining agents for soils, waterstops for civil engineering and construction, sheets for seeding, and goods for food distribution as well as hygienic goods such as paper diapers for babies since they started to put into practical use.

As the method for preparing a superabsorbent polymer, reverse phase suspension polymerization, aqueous solution polymerization, or the like is known. The reverse phase suspension polymerization is disclosed in JP-S-56-161408, JP-S-57-158209, and JP-S-57-198714. As the aqueous solution polymerization, thermopolymerization in which polymerization is performed by heating an aqueous solution, photopolymerization in which polymerization is performed by ultraviolet (UV) irradiation, and the like are known.

### DISCLOSURE

### TECHNICAL PROBLEM

An object to be achieved by the present invention is to improve the process efficiency by reprocessing the by-products generated in the manufacturing process.

However, objects of the present invention are not restricted to the one set forth herein. The above and other aspects of the present invention will become more apparent to one of ordinary skill in the art to which the present invention pertains by referencing the detailed description of the present invention given below.

### TECHNICAL SOLUTION

According to an exemplary embodiment of the invention to solve the technical problem, a method for preparing a superabsorbent polymer, comprising: polymerizing a monomer composition into a superabsorbent polymer in a polymerization reactor; pulverizing the superabsorbent polymer; and hydrolyzing fine particles generated during a process and reusing the hydrolyzed fine particles for the monomer composition.

The fine particles may have an average particle diameter of smaller than 300 µm.

The method may further comprise: cutting the polymer before the pulverizing of the polymer.

The method may further comprise: drying the polymer before the pulverizing of the polymer.

The method may further comprise: surface-crosslinking of surface of the superabsorbent polymer.

The method may further comprise: classifying the superabsorbent polymer by size.

A medium for hydrolyzing the fine particles may be an alkali solution.

The alkali solution may be an aqueous sodium hydroxide solution.

According to another exemplary embodiment of the invention to solve the technical problem, a method for preparing a superabsorbent polymer, comprising: polymerizing a monomer composition into a superabsorbent polymer in a polymerization reactor; pulverizing the superabsorbent polymer; and directly reusing fine particles generated during a process for the monomer composition.

Specific details of other embodiments are included in the detailed description and drawings.

### ADVANTAGEOUS EFFECTS

According to embodiments of the present invention, there are at least following effects.

Through the preparing method of the present invention, process efficiency can be improved, and raw material costs can be reduced.

The effects of the present invention are not limited to the above-described effects, and other various effects are anticipated herein.

### BEST MODE FOR INVENTION

Features of the invention and methods of accomplishing the same may be understood more readily by reference to the following detailed description of preferred embodiments and the accompanying drawings. The invention may, however, be embodied in many different forms and should not be construed as being limited to the embodiments set forth herein. Rather, these embodiments are provided so that this disclosure will be thorough and complete and will fully convey the concept of the invention to those skilled in the art, and the invention will only be defined by the appended claims. Like numbers refer to like elements throughout this specifications. In the drawings, the thickness of layers and regions are exaggerated for clarity.

It will be understood that, although the terms first, second etc., may be used herein to describe various elements or components should not be limited by these terms. These terms are only used to distinguish one element or component from another element or component. Thus, a first element or component discussed below could be termed a second element or component without departing from the teachings of the invention.

### Method for preparing a superabsorbent polymer

A method for preparing a superabsorbent polymer according to an embodiment of the present invention includes the steps of: polymerizing a monomer composition into a superabsorbent polymer in a polymerization reactor; pulverizing the superabsorbent polymer; and hydrolyzing the fine particles generated during the process and reusing the hydrolyzed fine particles for the monomer composition.

In the step of polymerizing the monomer composition, although not particularly limited, the monomer composition may be polymerized by injecting the monomer composition into a polymerization reactor. For the purpose of an efficient process, the monomer composition may be continuously polymerized by using a continuous polymerization reactor. In this case, in order to form a superabsorbent, the monomer composition may be polymerized by applying the monomer composition onto a belt, but the present invention is not limited thereto.

As the monomer contained in the monomer composition, a water-soluble unsaturated ethylene-based monomer can be used without limitation as long as it is generally used in the manufacture of a superabsorbent polymer. The monomer may include one or more selected from the group consisting of anionic monomers and salts thereof, non-ionic monomers having hydrophilicity, and unsaturated monomers containing an amino group and quaternary salts thereof.

In exemplary embodiment, examples of the monomer may include: on or more anionic monomers or salts thereof selected from the group consisting of acrylic acid, methacrylic acid, anhydrous maleic acid, fumaric acid, crotonic acid, itaconic acid, 2-acryloylethanesulfonic acid, 2-methacryloylethanesulfonic acid, 2-(meth)acryloylpropane sulfonic acid,and 2-(meth)acrylamide-2-methylpropane sulfonic acid; one or more non-ionic monomers having hydrophilicity selected from the group consisting of (meth)acrylamide, N-substituted (meth)acrylate, 2-hydroxyethyl (meth)acrylate, 2-hydroxypropyl (meth)acrylate, methoxy polyethylene glycol (meth)acrylate, and polyethylene glycol (meth)acrylate; and one or more unsaturated monomers containing an amino group selected from the group consisting of (N,N)-dimethylaminoethyl (meth)acrylate and (N,N)-dimethylaminopropyl (meth)acrylamide or quaternary salts thereof.

The concentration of the water-soluble unsaturated ethylene-based monomer in the monomer composition may be appropriately selected and used in consideration of polymerization time and reaction conditions (the feed rate of the monomer composition, the irradiation time, irradiation range and irradiation intensity of heat and/or light, and the width, length and moving speed of the belt). In exemplary embodiment, the concentration thereof may be in a range of 40 wt% to 60 wt%. In this case, it is efficient in terms of solubility of the monomer and economical aspects.

The monomer composition may further include at least one additive selected from the group consisting of a photopolymerization initiator, a thermopolymerization initiator, and a cross-linker. The polymerization initiator can be suitably selected from a photopolymerization initiator, a thermopolymerization initiator, and a photopolymerization initiator and a thermopolymerization initiator depending on the kind of polymerization.

The photopolymerization initiator is not particularly limited, but examples thereof may include, but are not limited to, acetophenone derivatives, such as diethoxyacetophenone, 2-hydroxy-2-methyl-1-phenylpropan-1-one, 4-(2-hydroxyethoxy)phenyl-(2-hydroxy)-2-propyl ketone, and 1-hydroxycyclohexyl phenyl ketone; benzoin alkyl ethers, such as benzoin methyl ether, benzoin ethyl ether, benzoin isopropyl ether, and benzoin isobutyl ether;benzophenone derivatives, such as methyl o-benzoyl benzoate, 4-phenyl benzophenone, 4-benzoyl-4'-methyl-diphenyl sulfide, and (4-benzoyl-benzyl)trimethyl ammonium chloride;thioxanthone-based compounds; acylphosphine oxide derivatives, such as bis(2,4,6-trimethylbenzoyl)-phenyl phosphine oxide and diphenyl (2,4,6-trimethylbenzoyl)-phosphine oxide; and azo compounds, such as 2-hydroxy-methyl-propionitrile and 2,2'-(azo-bis(2-methyl-N-(1,1'-bis(hydroxyme-thyl)-2-hydroxyethyl)propionamide). They may be used alone or as a mixture of two or more thereof.

The thermopolymerization initiator is not particularly limited, but examples thereof include an azo-based initiator, a peroxide-based initiator, a redox-based initiator, and an organic halide-based initiator. They may be used alone or as a mixture of two or more thereof. Specific examples of the thermopolymerization initiator may include, but are not limited to, sodium persulfate (Na₂S₂O₈) and potassium persulfate (K₂S₂O₈).

In the monomer composition, the content of each of the photopolymerization initiator and the thermopolymerization initiator can be selected and used, if effects of polymerization initiation can be exhibited. In exemplary embodiment, the photopolymerization initiator may be contained in the monomer composition in an amount of 0.005 to 0.1 parts by weight based on 100 parts by weight of the monomer, and the thermopolymerization initiator may be contained in the monomer composition in an amount of 0.01 to 0.5 parts by weight based on 100 parts by weight of the monomer, but the contents thereof are not limited thereto.

As the cross-linker, a cross-linker having at least one functional group and at least one unsaturated ethylenic group which can react with a substituent of the monomer, or a cross-linker having two or more functional groups which can react with a substituent of the monomer and/or a substituent formed by hydrolysis of the monomer can be used.

In exemplary embodiment, examples of the cross-linker may include bisacrylamide having 8 to 12 carbon atoms, bismethacrylamide having 8 to 12 carbon atoms, poly(meth)acrylate of a polyol having 2 to 10 carbon atoms, and poly(meth)allyl ether of a polyol having 2 to 10 carbon atoms. Specific examples of the cross-linker include, but are not limited to, N,N'-methylene bis(meth)acrylate, ethyleneoxy (meth)acrylate, polyethyleneoxy (meth)acrylate, propyleneoxy (meth)acrylate, glycerol diacrylate, glycerol triacrylate, trimethyloltriacrylate, triallylamine, triarylcyanurate, triallylisocyanate, polyethylene glycol, diethylene glycol, propylene glycol, and mixtures of two or more thereof.

In the monomer composition, the content of the cross-linker may be appropriately selected and used, if effects of cross-linkage can be exhibited. In exemplary embodiment, the cross-linker can be contained in an amount of 0.01 to 0.5 parts by weight, based on 100 parts by weight of the monomer, but the content thereof is not limited thereto.

The obtained superabsorbent polymer is put into a cutting machine, and is cut by a cutter.

The method for preparing a superabsorbent polymer may further include the step of pulverizing, drying and further pulverizing the dried polymer. In some cases, when the method for preparing a superabsorbent polymer further includes the step of preliminarily drying the superabsorbent polymer before the step of pulverizing the superabsorbent polymer, the aggregation of the superabsorbent polymer can be prevented.

In the method of pulverizing the superabsorbent polymer, although not particularly limited, for example, an apparatus for cutting and extruding a rubber-like elastic body may be used. In exemplary embodiment, examples thereof may include, but are not limited to, a blade cutter, a chop cutter,
a kneading cutter, a vibration pulverizer, an impact pulverizer, and a friction pulverizer.

In the method of drying the polymer, generally, a dryer and a furnace can be used. In exemplary embodiment, examples thereof may include, but are not limited to, a hot-air dryer, a fluid-bed dryer, a flash dryer, an infrared dryer, a dielectric heating dryer. Drying temperature is not particularly limited, but may be 100°C to 200°C in terms of thermal degradation prevention and efficient drying.

The method for preparing a superabsorbent polymer may further include the step of classifying the pulverized superabsorbent polymer particles by size. The size of the superabsorbent polymer particles may be suitably selected without limitation depending on the applications and characteristics of articles to be used. When the size of the superabsorbent polymer particles is too large, the physical properties of absorbent articles using the superabsorbent polymer particles may be deteriorated. In contrast, when the size thereof is too small, absorption capacity may be decreased, and fine particles generated during the process fly to do harm to workers, which is not preferable.

In the method of classifying the superabsorbent polymer particles, although not particularly limited, for example, a sieve, a dust collector, or the like may be used. Meanwhile, in addition to the classification of the superabsorbent polymer particles, fine particles can be separately classified by a dust collector during the entire process.

The size of fine particles is not particularly limited as long as the size thereof is smaller than the size of the superabsorbent polymer particles. For example, the average diameter of fine particles may be smaller than 300 µm. In some cases, the superabsorbent polymer particles having an average diameter of smaller than 150 µm may also be classified into fine particles.

The polymerization reaction of the separately classified fine particles may be performed again after the crosslinked portion of a crosslinked polymer is broken and dissolved in a medium for hydrolysis. In this case, since the polymerization reaction is performed in a state in which the crosslinkage of the fine particles is dissociated, the physical properties of after the polymerization are not greatly influenced.

Conventionally, an apparatus for reassembling fine particles by aggregating the fine particles using water has been used. In the apparatus, the reassembled fine particles are mixed with the gel obtained by polymerization during the process. However, since the reassembled fine particles are mechanically treated in this method, the reassembled fine particles easily are separated into fine particles during the process, and thus an apparatus for reassembling the fine particles is required, thereby increasing economic burden.

However, the present invention is advantageous in that the polymer particles are mixed with the reused fine particles before polymerization to form chemical bonds, and thus the polymer particles and the reused fine particles are not easily separated, and an additional apparatus is not required.

The medium for hydrolysis is not particularly limited as long as it can break a bond by hydrolyzing the crosslinked portion of a crosslinked polymer. For example, the medium for hydrolysis is an alkali solution, but is not limited thereto. In an exemplary embodiment, the alkali solution may be an aqueous sodium hydroxide solution, and the concentration of the aqueous sodium hydroxide solution may be in a range of 10% to 70%. However, the present invention is not limited thereto, and an aqueous sodium hydroxide solution having suitable concentration may also be selected depending on reaction time, temperature, or the like.

In an exemplary embodiment, the method for preparing a superabsorbent polymer may further include the step of crosslinking the surface of the superabsorbent polymer.

The surface crosslinking may be performed by using ethyleneglycol diglycidyl ether, water, and ethanol, but the present invention is not limited thereto.

The surface crosslinking may be carried out after forming particles through pulverizing and drying, but the present invention is not limited thereto. If necessary, the surface crosslinking may be carried out several times.

The classifying step may be applied even after the surface crosslinking step.

A method for preparing a superabsorbent polymer according to another embodiment of the present invention includes the steps of: polymerizing a monomer composition into a superabsorbent polymer in a polymerization reactor; pulverizing the superabsorbent polymer; and directly reusing the fine particles generated during the process for the monomer composition.

In the method, since the monomer composition is mixed with the fine particles before polymerization without hydrolysis, both the monomer composition and the fine particles are polymerized with each other, and thus the physical properties after the polymerization are not greatly influenced.

### MODE FOR INVENTION

### Comparative Example 1

84 g of 50% caustic soda was put into a reaction container, diluted with 98.3 g of water, stirred, and then neutralized by the addition of 107.7 g of acrylic acid. After the temperature increase due to heat of neutralization, the neutralization product was cooled to 40°C, mixed with 0.1 g of polyethylene glycol diacrylate, as an internal crosslinker, and 0.36 g of a 3% diphenyl(2,4,5-trimethylbenzoyl)-phosphine oxide solution, further mixed with 1.08 g of a 3% potassium sulfate solution, as a thermal initiator, and then polymerized by a UV lamp having an intensity of 8 mW/cm² for 3 minutes, so as to obtain a gel-like polymer. After the polymerization,
the obtained gel-like polymer was cut by a chopper, dried in a hot air oven at 180°C for 1 hour to make the polymer hard polymer, pulverized by a pulverizing machine, classified at a size of 150 to 850 µm, and then physical properties were measured.

### Example 1

84 g of 50% caustic soda and 2.16 g of fine particles having a size of 150 µm or less were put into a reaction container, stirred for 2 hours, mixed with 98.3 g of water, stirred, and then neutralized by the addition of 107.7 g of acrylic acid. After the temperature increase due to heat of neutralization, the neutralization product was cooled to 40°C, mixed with 0.1 g of polyethylene glycol diacrylate, as an internal crosslinker, and 0.36 g of a 3% diphenyl(2,4,5-trimethylbenzoyl)-phosphine oxide solution, further mixed with 1.08 g of a 3% potassium sulfate solution, as a thermal initiator, and then polymerized by a UV lamp having an intensity of 8 mW/cm² for 3 minutes, so as to obtain a gel-like polymer. After the polymerization, the obtained gel-like polymer was cut by a chopper, dried in a hot air oven at 180°C for 1 hour to make the polymer hard polymer, pulverized by a pulverizing machine, classified at a size of 150 to 850 µm, and then physical properties were measured.

### Example 2

84 g of 50% caustic soda and 98.3 g of water were put into a reaction container, stirred, and then neutralized by the addition of 107.7 g of acrylic acid. After the temperature increase due to heat of neutralization, the neutralization product was cooled to 40°C, mixed with 0.1 g of polyethylene glycol diacrylate, as an internal crosslinker, and 0.36 g of a 3% diphenyl(2,4,5-trimethylbenzoyl)-phosphine oxide solution, further mixed with 1.08 g of a 3% potassium sulfate solution, as a thermal initiator, and then polymerized by a UV lamp having an intensity of 8 mW/cm² for 3 minutes, so as to obtain a gel-like polymer. After the polymerization, the obtained gel-like polymer was cut by a chopper, dried in a hot air oven at 180°C for 1 hour to make the polymer hard polymer, pulverized by a pulverizing machine, classified at a size of 150 to 850 µm, and then physical properties were measured.

### Experimental Example 1

The CRCs and ECs of superabsorbent polymers obtained in Comparative Example 1 and Examples 1 and 2 were measured, and the results thereof are summarized in Table 1 below.

**[Table 1]**

| | CRC | EC |
|---|---|---|
| Comparative Example 1 | 54 | 11 |
| Example 1 | 54 | 9 |
| Example 2 | 56 | 11 |

Referring to Table 1, it can be ascertained that the CRC of the superabsorbent of Example 1 is equal to that of the superabsorbent of Comparative Example 1, and the EC of the superabsorbent of Example 1 is lower than that of the superabsorbent of Comparative Example 1. Therefore, in absorption capacity, the superabsorbent polymer of Comparative Example 1 and the superabsorbent polymer of Example 1 can be maintained at the same level, and, in gel blocking, the superabsorbent polymer of Example 1 is more excellent than the superabsorbent polymer of Comparative Example 1 because the EC of the superabsorbent polymer of Example 1 is lower than that of the superabsorbent polymer of Comparative Example 1.

The reason for this is that the fine particles of Example 1 serve as a crosslinker during polymerization, so as to lower the EC of the superabsorbent polymer. In some cases, it is possible to prepare a superabsorbent polymer having physical properties similar to that of the superabsorbent polymer of Comparative Example 1 by forming the crosslinker into fine particles.

Further, it can be ascertained that the CRC of the superabsorbent of Example 2 is higher than those of the superabsorbent polymer of Comparative Example 1 and the superabsorbent of Example 1, and thus the superabsorbent polymer of Example 2 has high absorption capacity, and the EC of the superabsorbent polymer of Example 2 is maintained at the same level as that of the superabsorbent polymer of Comparative Example 1. Therefore, it can be ascertained that it is possible to prepare a superabsorbent polymer even though polymerization is performed by the addition of fine particles without additional neutralization treatment.

While exemplary embodiments have been shown and described above, it will be apparent to those skilled in the art will appreciate that many variations and modifications can be made to the exemplary embodiments without substantially departing from the spirit of the present invention. Therefore, the disclosed exemplary embodiments of the invention should be considered in a generic and descriptive sense only and not for purposes of limitation.

## Claims

1. A method for preparing a superabsorbent polymer, comprising:
polymerizing a monomer composition into a superabsorbent polymer in a polymerization reactor;
pulverizing the superabsorbent polymer; and
hydrolyzing fine particles generated during a process and
reusing the hydrolyzed fine particles for the monomer composition.

2. The method of claim 1,
wherein the fine particles have an average particle diameter of smaller than 300 µm.

3. The method of claim 1, further comprising:
cutting the polymer before the pulverizing of the polymer.

4. The method of claim 1, further comprising:
drying the polymer before the pulverizing of the polymer.

5. The method of claim 1, further comprising:
surface-crosslinking of surface of the superabsorbent polymer.

6. The method of claim 1, further comprising:
classifying the superabsorbent polymer by size.

7. The method of claim 1,
wherein a medium for hydrolyzing the fine particles is an alkali solution.

8. The method of claim 7,
wherein the alkali solution is an aqueous sodium hydroxide solution.

9. A method for preparing a superabsorbent polymer, comprising:
polymerizing a monomer composition into a superabsorbent polymer in a polymerization reactor;
pulverizing the superabsorbent polymer; and
directly reusing fine particles generated during a process for the monomer composition.
